# EUROPEAN PATENT SPECIFICATION

(11) **EP 4 158 336 B1**
(45) Date of publication and mention of the grant of the patent: **25.06.2025**
(21) Application number: 21729832.2
(22) Date of filing: 26.05.2021
(51) Int. Cl.: G01N 33/487

(54) **NANOPORE CELL WITH SEAMLESS WORKING ELECTRODE AND METHODS OF FORMING THE SAME**
NANOPORENZELLE MIT NAHTLOSER ARBEITSELEKTRODE UND VERFAHREN ZUR FORMUNG DAVON
CELLULE À NANOPORES AVEC ÉLECTRODE DE TRAVAIL SANS DISCONTINUITÉ ET PROCÉDÉS POUR SA FORMATION

(30) Priority: 26.05.2020 US 202063029936 P
(43) Date of publication of application: 05.04.2023
(73) Proprietor: F. Hoffmann-La Roche AG, 4070 Basel (CH); Roche Diagnostics GmbH, 68305 Mannheim (DE)
(72) Inventor: FOSTER, John C., Pleasanton, California 4300 (US); HONER, Kenneth A., Pleasanton, California 4300 (US); NG, Marowen, Pleasanton, California 4300 (US)
(74) Representative: Merkel, Patrick
(86) International application number: PCT/EP2021/063983
(87) International publication number: WO 2021/239781

(56) References cited:
- US-A1- 2018 266 980

## Description

### CROSS REFERENCE TO RELATED APPLICATIONS

This application claims priority to U.S. Provisional Application No. 63/029,936, filed May 26, 2020.

### BACKGROUND

Nanopore membrane devices having pore sizes on the order of one nanometer in internal diameter have shown promise in rapid nucleotide sequencing. When a voltage potential is applied across a nanopore immersed in a conducting fluid, a small ion current attributed to the conduction of ions across the nanopore can exist. The size of the current is sensitive to the pore size and which molecule is in the nanopore. The molecule can be a nucleotide itself (e.g., as part of a nucleic acid) or a particular tag attached to a particular nucleotide, thereby allowing detection of a nucleotide at a particular position of a nucleic acid. A voltage in a circuit including the nanopore can be measured (e.g., at an integrating capacitor) as a way of measuring the resistance of the molecule, thereby allowing detection of which molecule is in the nanopore.

Even though nanopore-based sequencing sensor chips have been successful in some applications, improvements are still desirable. For example, there is a need for improved nanopore well structures and methods. In some cases, it has been found that working electrodes of nanopore-based sequencing sensor chips are preferably made from porous electrode material to maximize capacitance and surface area. Because reliable porous working electrodes with desired characteristics (such as wettability and adequate capacitance) are critical for the operation of nanopore-based sequencing devices, methods of protecting the working electrode during manufacture are implemented. In particular, the working electrode must be protected while hydrophobic cladding is deposited and patterned to form the well of the nanopore cell. Some methods of protecting porous working electrodes include application of a protective layer (such as a dielectric layer) to be used as a buffer layer or sacrificial layer to protect a porous working electrode during subsequent steps. In many cases, it is desirable to easily remove the protective layer by chemical processes, thus exposing the porous working electrode for operation. However, the chemicals used to remove the protective layers can potentially attack other layers if not accounted for in the structure and process of forming the porous electrode and the nanopore cell.

US 2018/266980 discloses a nanopore-based sequencing chip with a substrate having a conductive layer disposed in a top portion of the substrate and a dielectric layer surrounding the conductive layer, which can be connected to a circuit. US 2018/266980 also discloses a process for producing such a chip where a sacrificial metal layer is used for protecting a porous working electrode during cell manufacturing. During this manufacture, the layer of dielectric is etched to create an opening to expose a top surface of conductive layer. The porous electrode layer is then formed on the conductive layer in this opening of a first dielectric layer. The porous electrode can be a porous TiN layer and can be grown in a manner to create rough, sparsely-spaced columnar structures or columns of crystals that provide a high specific surface area that can come in contact with an electrolyte.

### BRIEF SUMMARY

Accordingly, nanopore cells and methods for forming nanopore cells are described in accordance with embodiments of the present disclosure. The nanopore cells and method for forming the nanopore cells ensure that the working electrodes are structurally sound so that the chemical removal of protective layers does not negatively affect other components of the nanopore cell. In particular, the nanopore cells and methods for forming the nanopore cells ensure that the working electrodes are formed with seamless columns of porous materials, which reduces the likelihood of chemicals seeping into and damaging other layers of the nanopore cell.

Embodiments are directed to a method for forming a nanopore cell. The method of the present invention includes providing a device structure comprising a conductive layer disposed on a top portion of a substrate and an interconnect dielectric layer overlying the conductive layer. The method includes removing a portion of the interconnect dielectric layer to form a planar electrode support surface. The planar electrode support surface includes an exposed island of the conductive layer surrounded by a remaining portion of the interconnect dielectric layer. The method further includes depositing a porous electrode material on the planar electrode support surface to form a seamless porous electrode layer. The seamless porous electrode layer includes columns of the porous electrode material. The method further includes depositing a protective layer on the seamless porous electrode layer, patterning the seamless porous electrode layer and the protective layer to a form a working electrode island, depositing and patterning a hydrophobic cladding on the working electrode island to form the sidewalls of a well of the nanopore cell, and removing at least a portion of the protective layer to expose the porous electrode layer. The exposed porous electrode layer forms at least a portion of a bottom wall of the well of the nanopore cell.

Some embodiments may include a nanopore cell. The nanopore cell of the present invention includes a substrate, an electrode support layer overlying a top portion of the substrate, and a well. The electrode support layer includes a conductive layer island surrounded by an interconnect dielectric layer and a planar top surface formed by the conductive layer island and the interconnect dielectric layer. The well includes a seamless porous working electrode island disposed on the planar top surface of the electrode support layer, hydrophobic cladding surrounding the seamless porous working electrode island and patterned to form sidewalls of the well, and a cavity formed by the hydrophobic cladding and the seamless porous working electrode island. The seamless porous working electrode island includes columns of a porous electrode material. In some embodiments, the seamless working electrode island further includes a protective layer disposed on the columns of porous electrode material, wherein the protective layer is configured to be selectably removable to expose the porous electrode material to the cavity.

A better understanding of the nature and advantages of embodiments of the present invention may be gained with reference to the following detailed description and the accompanying drawings.

### BRIEF DESCRIPTION OF THE DRAWINGS

FIG. 1 illustrates an embodiment of a cell in a nanopore-based sequencing chip.
FIG. 2 illustrates an embodiment of a cell performing nucleotide sequencing with the Nano-SBS technique.
FIG. 3 illustrates an embodiment of an electrochemical cell of a nanopore-based sequencing chip that includes a TiN working electrode with increased electrochemical capacitance.
FIG. 4 illustrates an additional embodiment of a circuitry in a cell of a nanopore-based sequencing chip, wherein the voltage applied across the nanopore can be configured to vary over a time period during which the nanopore is in a particular detectable state.
FIG. 5 illustrates a double layer that is formed at any interface between a conductive electrode and an adjacent liquid electrolyte.
FIG. 6 illustrates a pseudocapacitance effect that can be formed, simultaneously with the formation of a double-layer, at an interface between a conductive electrode and an adjacent liquid electrolyte.
FIGS. 7A-7G (outside the scope of the claimed invention) illustrate an example of a process for constructing an electrochemical cell of a nanopore-based sequencing chip that includes a TiN working electrode using chemical mechanical planarization.
FIGS. 8A-8F (outside the scope of the claimed invention) illustrate an example of a process for constructing an electrochemical cell of a nanopore-based sequencing chip that includes a TiN working electrode using photolithography and dry etching.
FIG. 9 illustrates seams that may form in working electrodes constructed using the processes illustrated in FIGS. 7A-7G and 8A-8F.
FIG. 10 is a micrograph illustrating a TiN electrode layer formed using the process illustrated in FIGS. 7A-7G.
FIG. 11 is a micrograph illustrating a TiN electrode layer formed using the process illustrated in FIGS. 8A-8F.
FIGS. 12A-12F illustrate an embodiment of a process for constructing an electrochemical cell of a nanopore-based sequencing chip that includes a seamless working electrode.
FIG. 13 illustrates the seamless electrodes constructed using the process illustrated in FIGS. 12A-12F.
FIGS. 14A and 14B are micrographs of an example of an array of seamless electrodes formed by the method of FIGS. 12A-12F.

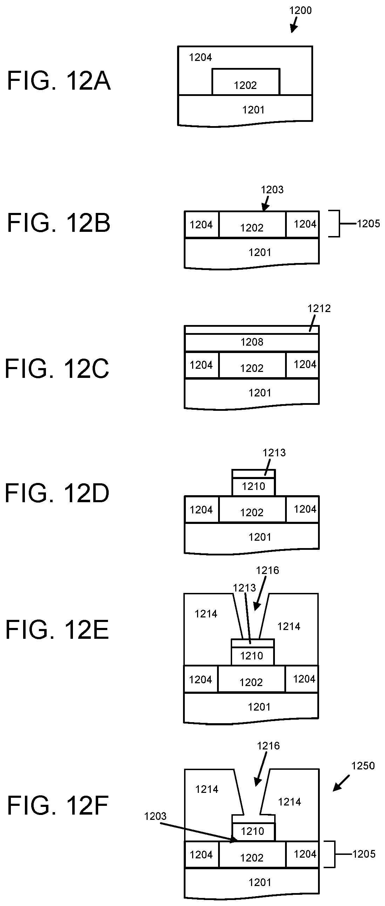

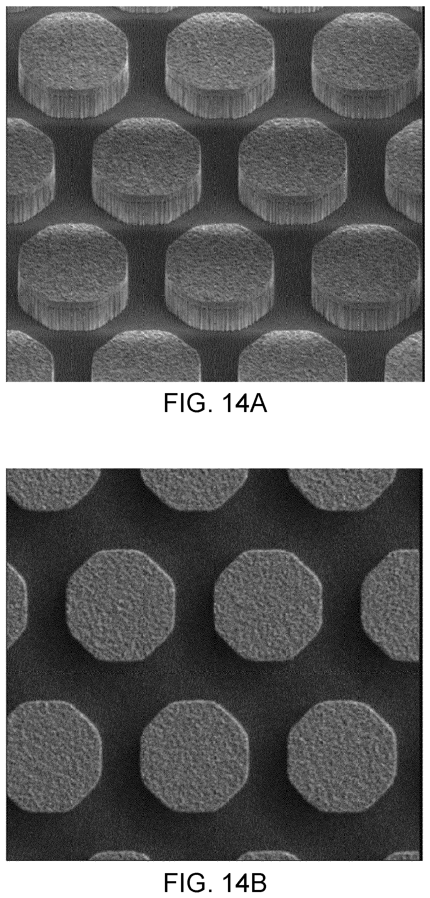

FIGS. 14A and 14B are micrographs of an example of an array of seamless electrodes formed by the method of FIGS. 12A-12F.

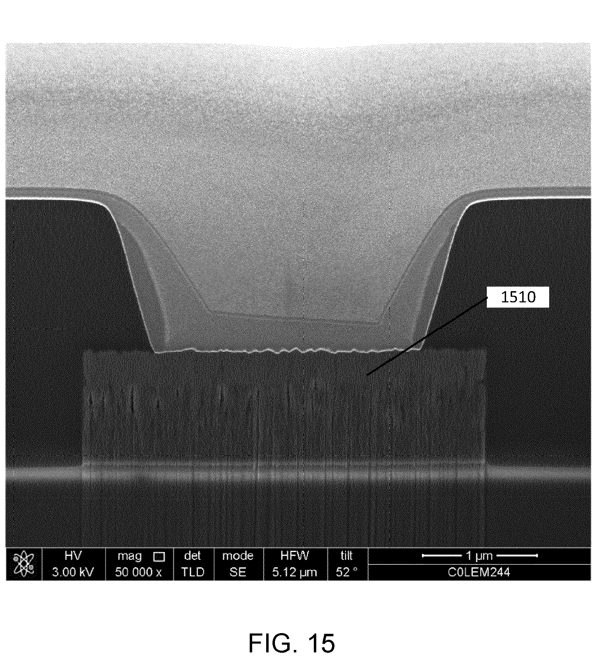

FIG. 15 is a micrograph of an example of a seamless TiN electrode layer formed by the method of FIGS. 12A-12F.

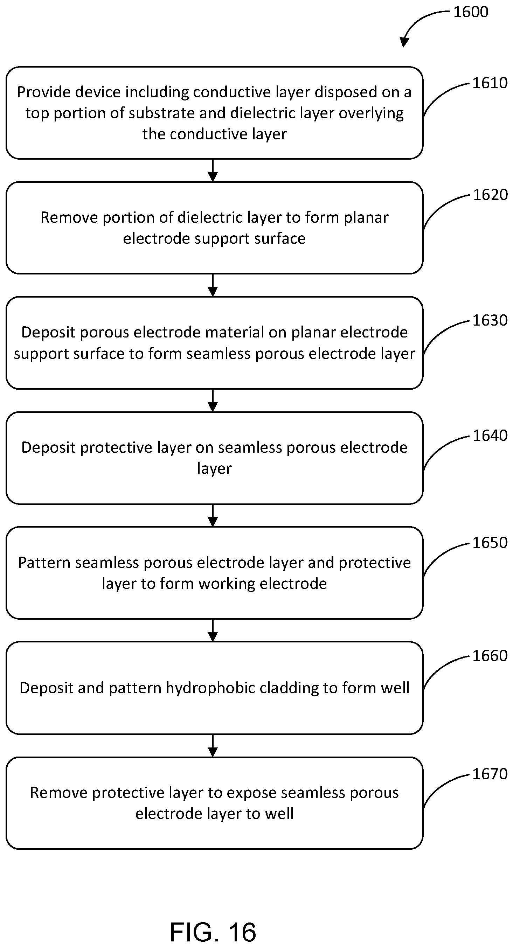

FIG. 16 is a flowchart illustrating a method for forming a nanopore cell with a seamless electrode.

### TERMS

*A "nanopore"* refers to a pore, channel or passage formed or otherwise provided in a membrane. A membrane can be an organic membrane, such as a lipid bilayer, or a synthetic membrane, such as a membrane formed of a polymeric material. The nanopore can be disposed adjacent or in proximity to a sensing circuit or an electrode coupled to a sensing circuit, such as, for example, a complementary metal oxide semiconductor (CMOS) or field effect transistor (FET) circuit. In some examples, a nanopore has a characteristic width or diameter on the order of 0.1 nanometers (nm) to about 1000 nm. Some nanopores are proteins.

A *"well"* in a nanopore device refers to a structure formed by insulating walls and a working electrode into which an electrolyte may be contained. A *"well profile"* refers to a structural description of the well and can include measures of an angle and a sharpness of a well edge. A "cell" of a nanopore device can include at various stages of operation: a well, a nanopore (e.g., in a membrane across the well), and a working electrode, as well as other circuitry, e.g., data acquisition circuitry.

*A "dielectric material"* refers to an electrical insulator that can be polarized by an applied electric field. When a dielectric is placed in an electric field, electric charges do not flow through the material as they do in a conductor, but only slightly shift from their average equilibrium positions causing dielectric polarization. A *"conductive layer"* refers to a layer of material that allows the flow of an electrical current in one or more directions. A metal wire is a common electrical conductor.

*A "porous material"* refers to a material that contains pores or voids at a surface of the material. A *"spongy material"* refers to a material having an open, porous structure.

### DETAILED DESCRIPTION

In a nanopore device, a membrane can be formed over a well in a dielectric layer. For example, the membrane can include a lipid monolayer formed on top of the dielectric layer. As the membrane reaches the opening of well, the lipid monolayer can transition to a lipid bilayer that spans across the opening of the well. The structure of the well and the materials that form the well can perform important roles in the formation of the membrane and the insertion of the nanopore in the membrane, and the interaction between the materials forming the well can also affect the operation of the nanopore device.

The description below includes an overview of a structure and operation of nanopore cells. The impact of the structure of the well, the materials that form the well, and the interaction thereof are also discussed. The problems caused by the interaction between chemicals used to remove protective layers and a porous working electrode are also described, along with proposed solutions.

### I. OVERVIEW OF NANOPORE CELLS

This section includes an introduction to the operation of a nanopore cell, cell structure and usage, and circuitry for measuring signal. The capacitive effects at a working electrode (referred to as a double layer capacitance) are explained, and example processes of constructing a porous working electrode are described.

### A. Operation of the cell

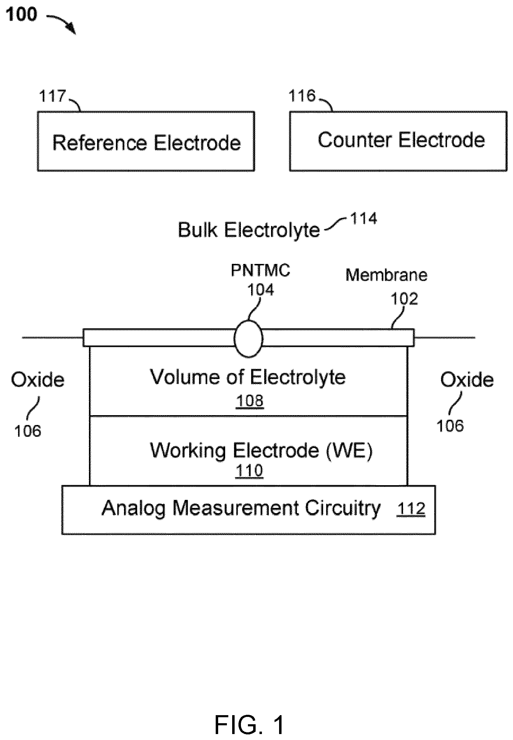

FIG. 1 illustrates an embodiment of a cell 100 in an array of cells that form a nanopore-based sequencing chip. A membrane 102 is formed over the surface of the cell. In some embodiments, membrane 102 is a lipid bilayer. The bulk electrolyte 114 containing protein nanopore transmembrane molecular complexes (PNTMC) and the analyte of interest (e.g., a single polymer molecule, such as DNA) can be placed directly onto the surface of the cell. A single PNTMC 104 can be inserted into membrane 102 by electroporation. The individual membranes in the array are neither chemically nor electrically connected to each other. Thus, each cell in the array is an independent sequencing machine, producing data unique to the single polymer molecule associated with the PNTMC. PNTMC 104 can modulate the ionic current through the otherwise impermeable bilayer.

Analog measurement circuitry 112 is connected to a working electrode 110 (e.g., made of metal) covered by a volume of electrolyte 108 inside a well formed in an oxide layer 106. The volume of electrolyte 108 is isolated from the bulk electrolyte 114 by the ion-impermeable membrane 102. PNTMC 104 crosses membrane 102 and provides the only path for ionic current to flow from the bulk liquid to working electrode 110. The cell also includes a counter electrode (CE) 116. The cell also includes a reference electrode 117, which can act as an electrochemical potential sensor.

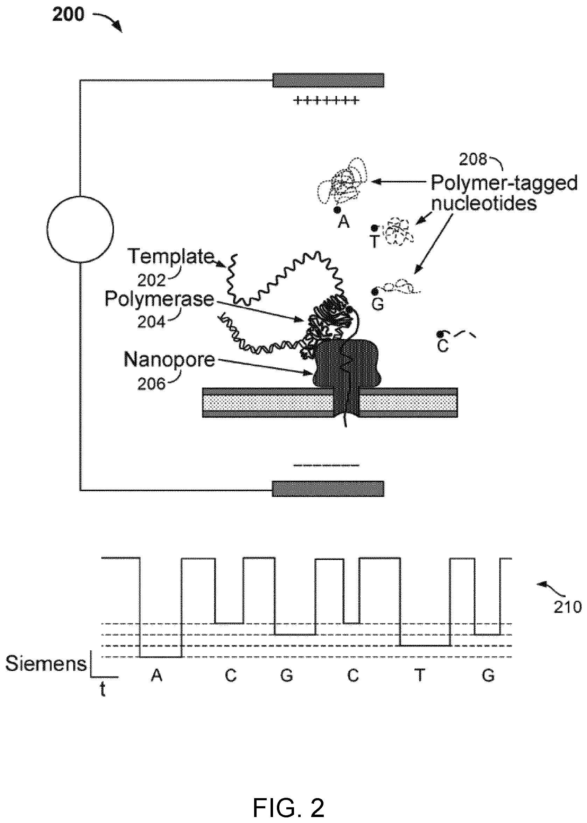

FIG. 2 illustrates an embodiment of a cell 200 performing nucleotide sequencing with the nanopore-based sequencing by a synthesis (Nano-SBS) technique. In the Nano-SBS technique, a template 202 to be sequenced and a primer are introduced to cell 200. To this template-primer complex, four differently tagged nucleotides 208, A, T, G, and C are added to the bulk aqueous phase. As the correctly tagged nucleotide is complexed with the polymerase 204, the tail of the tag is positioned in the barrel of nanopore 206. The tag held in the barrel of nanopore 206 generates a unique ionic blockade signal 210, thereby electronically identifying the added base due to the tags' distinct chemical structures.

### B. Cell structure and usage

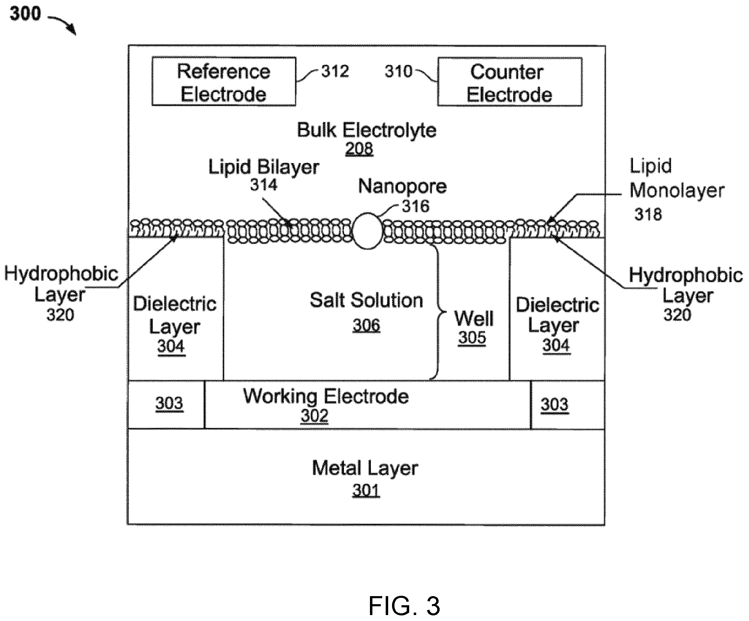

FIG. 3 illustrates an embodiment of electrochemical cell 300 of a nanopore-based sequencing chip that includes a working electrode (e.g., TiN, which has a high electrochemical capacitance). Cell 300 includes a conductive or metal layer 301. Metal layer 301 connects cell 300 to the remaining portions of the nanopore-based sequencing chip. In some embodiments, metal layer 301 is the top metal of the CMOS chip (e.g., metal 6 layer M6 of the underlying circuitry). Cell 300 further includes a working electrode 302 and a dielectric layer 303 above metal layer 301. In some embodiments, working electrode 302 can be circular or octagonal in shape, and dielectric layer 303 forms the walls surrounding working electrode 302. Cell 300 further includes a dielectric layer 304 above working electrode 302 and dielectric layer 303. Dielectric layer 304 forms the insulating walls surrounding a well 305.

In some embodiments, dielectric layer 303 and dielectric layer 304 together form a single piece of dielectric. Dielectric layer 303 is the portion that is disposed horizontally adjacent to working electrode 302, and dielectric layer 304 is the portion that is disposed above and covering a portion of the working electrode. In some embodiments, dielectric layer 303 and dielectric layer 304 are separate pieces of dielectric and they may be formed separately. Well 305 has an opening above an uncovered portion of the working electrode. In some embodiments, the opening above the uncovered portion of the working electrode can be circular or octagonal in shape.

Inside well 305, a volume of salt solution/electrolyte 306 is disposed above working electrode 302. Salt solution 306 may include one of the following: lithium chloride (LiCl), sodium chloride (NaCl), potassium chloride (KCl), lithium glutamate, sodium glutamate, potassium glutamate, lithium acetate, sodium acetate, potassium acetate, calcium chloride (CaClz), strontium chloride (SrClz), manganese chloride (MnClz), and magnesium chloride (MgClz). In some embodiments, salt solution 306 has a thickness of about three microns (µm). The thickness of salt solution 306 may range from 0 - 5 microns.

The dielectric material used to form dielectric layers 303 and 304 includes glass, oxide, silicon mononitride (SiN), and the like. The top surface of dielectric layer 304 may be silanized. Silanization forms a hydrophobic layer 320 above the top surface of dielectric layer 304. In some embodiments, hydrophobic layer 320 has a thickness of about 1.5 nanometers (nm). Alternatively, dielectric material that is hydrophobic such as hafnium oxide may be used to form dielectric layer 304.

As shown in FIG. 3, a membrane is formed on top of dielectric layer 304 and spans across well 305. For example, the membrane includes a lipid monolayer 318 formed on top of hydrophobic layer 320 and as the membrane reaches the opening of well 305, the lipid monolayer transitions to a lipid bilayer 314 that spans across the opening of the well. Hydrophobic layer 320 facilitates the formation of lipid monolayer 318 above dielectric layer 304 and the transition from a lipid monolayer to a lipid bilayer. A bulk electrolyte 308 containing protein nanopore transmembrane molecular complexes (PNTMC) and the analyte of interest is placed directly above the well. A single PNTMC/nanopore 316 is inserted into lipid bilayer 314 by electroporation. Nanopore 316 crosses lipid bilayer 314 and provides the only path for ionic flow from bulk electrolyte 308 to working electrode 302. Bulk electrolyte 308 may further include one of the following: lithium chloride (LiCl), sodium chloride (NaCl), potassium chloride (KCl), lithium glutamate, sodium glutamate, potassium glutamate, lithium acetate, sodium acetate, potassium acetate, calcium chloride (CaCb), strontium chloride (SrCb), manganese chloride (MnCb), and magnesium chloride (MgCb).

Cell 300 includes a counter electrode (CE) 310. Cell 300 also includes a reference electrode 312, which acts as an electrochemical potential sensor. In some embodiments, counter electrode 300 can be shared between a plurality of cells, and is therefore also referred to as a common electrode. The common electrode can be configured to apply a common potential to the bulk liquid in contact with the nanopores in the measurements cells. The common potential and the common electrode are common to all of the measurement cells.

Working electrode 302 is a titanium nitride (TiN) working electrode with increased electrochemical capacitance. The electrochemical capacitance associated with working electrode 302 may be increased by maximizing the specific surface area of the electrode. The specific surface area of working electrode 302 is the total surface area of the electrode per unit of mass (e. g., m²/kg) or per unit of volume (e. g., m²/m³, m²/m³, or m⁻¹ or per unit of base area (e. g., m²/m²). As the surface area increases, the electrochemical capacitance of the working electrode increases, and a greater amount of ions can be displaced with the same applied potential before the capacitor becomes charged. The surface area of working electrode 302 may be increased by making the TiN electrode "spongy" or porous. The TiN sponge soaks up electrolyte and creates a large effective surface area in contact with the electrolyte. The techniques for making and using a TiN is described further in U.S. Patent No. 10,174,371 to Foster et. al.

Other materials that can be used to form the working electrode include ruthenium, as described further in International Patent Publication WO2020043653A2 to Au et al.

The ratio of the capacitance associated with the membrane (C_{membrane}) and the capacitance associated with the working electrode (C_{electrochemical}) may be adjusted to achieve optimal overall system performance. Increased system performance may be achieved by reducing C_{membrane} while maximizing C_{electrochemical}. C_{membrane} can be adjusted to create the required RC time constant without the need for additional on-chip capacitance, thereby allowing a significant reduction in cell size and chip size.

In cell 300, the base surface area of the opening of well 305 (which is the same as the base surface area of lipid bilayer 314) and the base surface area of working electrode 302 are determined by the dimensions of dielectric layer 304 and dielectric layer 303, respectively. The base surface area of working electrode 302 is greater than or equal to the base surface area of the opening of well 305. Therefore, the two base surface areas may be optimized independently to provide the desired ratio between C_{membrane} and C_{electrochemical}. As shown in FIG. 3, a portion of working electrode 302 is covered by dielectric 304 and therefore the covered portion does not have direct contact with salt solution/electrolyte 306. By using a spongy and porous TiN working electrode, the electrolyte can diffuse through the spaces between the columnar TiN structures and vertically down the uncovered portion of the working electrode and then horizontally to the covered portion of working electrode 302 that is underneath dielectric layer 304. As a result, the effective surface area of TiN that is in contact with the electrolyte is maximized and C_{electrochemical} is maximized.

### C. Circuitry for measuring signal

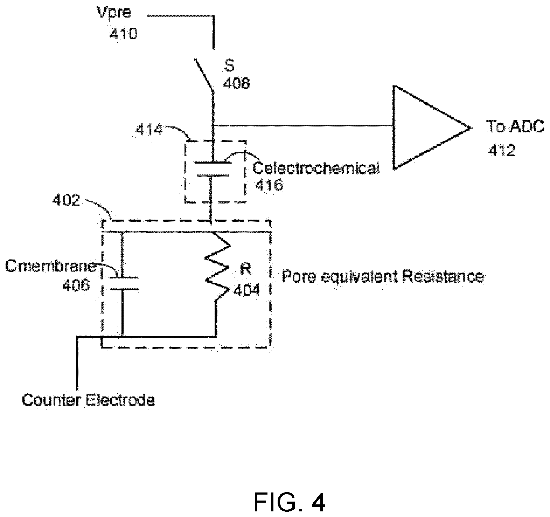

FIG. 4 illustrates an embodiment of a circuitry 400 in a cell of a nanopore-based sequencing chip, wherein the voltage or current applied across the nanopore can be configured to vary over a time period during which the nanopore is in a particular detectable state. In FIG. 4, instead of showing a nanopore inserted in a membrane and the liquid surrounding the nanopore, an electrical model 402 represents the electrical properties of the nanopore and the membrane, and an electrical model 414 represents the electrical properties of the working electrode are shown.

Electrical model 402 includes a capacitor 406 that models a capacitance associated with the membrane (C_{membrane}) and a resistor 404 that models a resistance associated with the nanopore in different states (e. g., the open-channel state or the states corresponding to having different types of tags or molecules inside the nanopore). Electrical model 414 includes a capacitor 416 that models a capacitance associated with the working electrode. The capacitance associated with the working electrode is also referred to as an electrochemical capacitance (C_{electrochemical}). The electrochemical capacitance C_{electrochemical} associated with the working electrode includes a double-layer capacitance and may further include a pseudo capacitance.

FIG. 4 also includes a switch 408 coupled to a voltage 410, which can be switched on and off for the purpose of measuring resistance 404. In some embodiments, voltage 410 is applied to electrical model 402 representing the nanopore. After capacitor 406 is fully charged (which may not be very long as it is desirable for the membrane to have a low capacitance), the switch 408 can be opened, and current can flow from one side of capacitor 406 to the other side via resistor 404 (i.e., the nanopore that includes the molecule being detected). Different values for resistor 404 will cause different current to flow, and thus different voltage decays. Capacitor 416 can be sufficiently large to not impact the circuit significantly.

After a specified amount of time, a voltage can be measured at an ADC (Analog-to-Digital Converter) 412. This can measure the time constant in the circuit represented by RC_{membrane}, as the voltage changes after the specified amount of time will correlate to the resistance of the pore (and thus the molecule inside of it). Embodiments can also measure an amount of time to reach a specific voltage, e.g., by using a comparator, as is described in U.S. Patent No. 9,377,437.

### D. Capacitive effects at working electrode (Double Layer Capacitance)

It is desirable for the working electrode to have a high capacitance, thereby reducing its impedance effect on the circuit, which can cause voltage levels to move slightly as a result of charge build up after multiple measurements that involve switch 408 opening and closing.

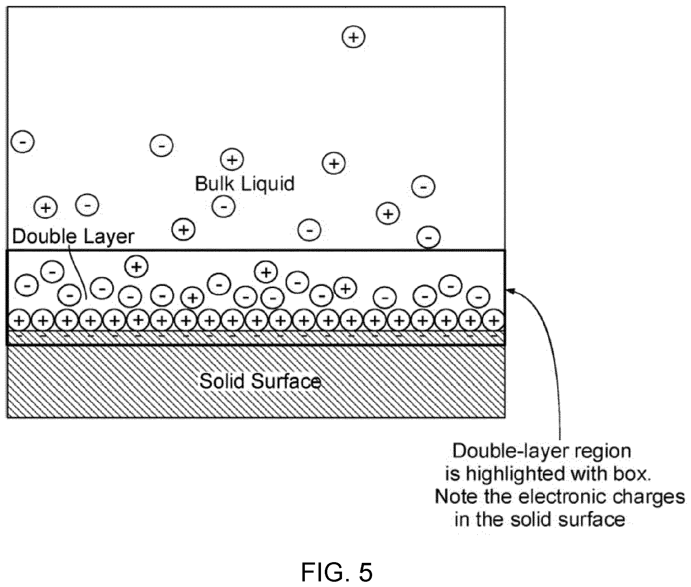

FIG. 5 illustrates a double layer that is formed at an interface between a conductive electrode and an adjacent liquid electrolyte. An electrical model for the double layer is shown as electrical model 414 in FIG. 4 that models a capacitance associated with the working electrode. In the example shown, the electrode surface is negatively charged, resulting in the accumulation of positively charged species in the electrolyte. In another example, the polarity of all charges may be opposite to the example shown. The charge in the electrode is balanced by reorientation of dipoles and accumulation of ions of opposite charge in the electrolyte near the interface. The accumulation of charges on either side of the interface between electrode and electrolyte, separated by a small distance due to the finite size of charged species and solvent molecules in the electrolyte, acts like a dielectric in a conventional capacitor. The term "double layer" refers to the ensemble of electronic and ionic charge distribution in the vicinity of the interface between the electrode and electrolyte.

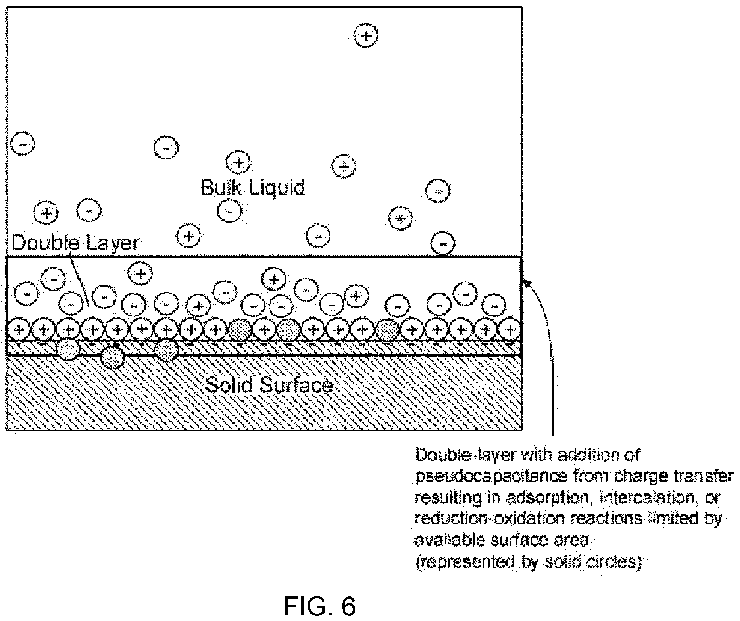

FIG. 6 illustrates a pseudocapacitance effect that can be formed, simultaneously with the formation of a double-layer as in FIG. 5, at an interface between a conductive electrode and an adjacent liquid electrolyte. FIG. 6 shows a double-layer with the addition of pseudo capacitance from charge transfer resulting in adsorption, intercalation, or reduction-oxidation reactions limited by available surface area (represented by solid circles).

### E. Example processes of constructing porous working electrode

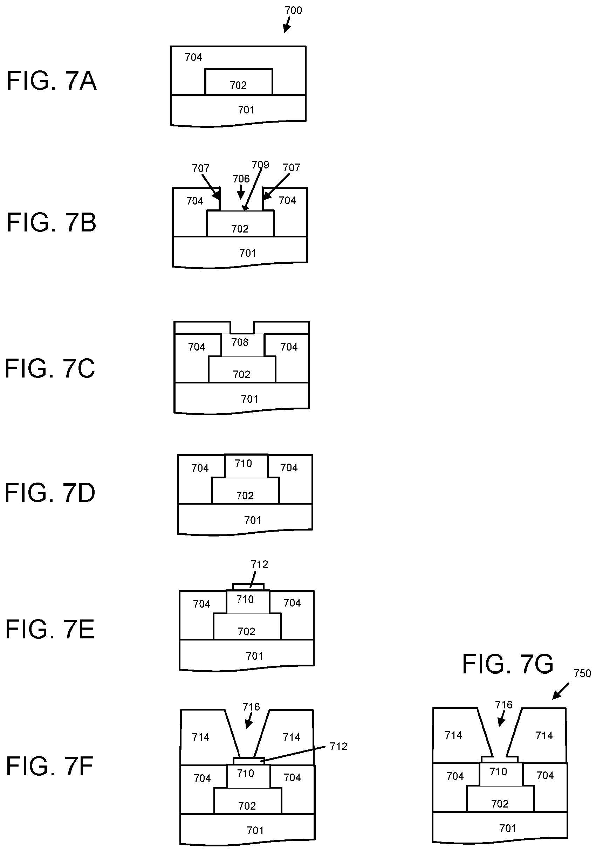

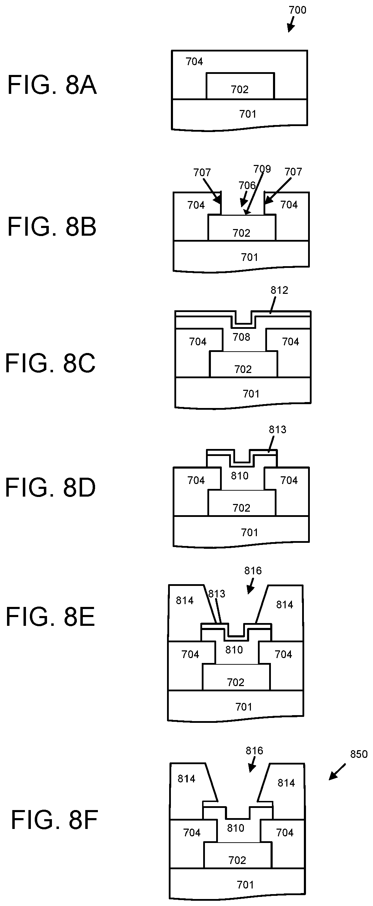

FIGS. 7A-7G and 8A-8F (not of the present invention) illustrate examples of processes for constructing an electrochemical cell of a nanopore-based sequencing chip that includes a TiN working electrode. Similar processes are described, for example, in U.S. Patent Application Serial No. 15/920,158, filed on March 18, 2018, the contents of which are incorporated in its entirety herein. In particular, FIGS. 7A-7G illustrate an example of a process for constructing an electrochemical cell of a nanopore-based sequencing chip that includes a TiN working electrode using chemical mechanical planarization, and FIGS. 8A-8F illustrate an example of a process for constructing an electrochemical cell of a nanopore-based sequencing chip that includes a TiN working electrode using photolithography and dry etching. As will be described in more detail below, while the processes result in working electrodes with desired spongy and porous characteristics, both of these methods can result in the formation of seams in the working electrode layers, which act as weak points and cause performance issues.

With reference to FIG. 7A, incoming material 700 may be provided for constructing the electrochemical nanopore cell. Incoming material 700 may include substrate 701, conductive layer 702, and dielectric layer 704 disposed thereon. Substrate 701, may be, for example, a CMOS substrate that includes circuitry for controlling the operation of the nanopore cell. Conductive layer 702 may be a part of the circuitries that deliver the signals from the cell to the rest of the chip. In some cases, conductive layer 702 can be the top metal layer of the circuitry, for example, the sixth metal layer. However, conductive layer 702 is not limited to being the sixth metal layer of the underlying circuitry. In some cases, conductive layer 702 can be an aluminum layer, for example, aluminum interconnect metal associated with the substrate. As shown in FIG. 7A, conductive layer 702 is enclosed in a layer of dielectric 704 (*e.g.,* SiO₂), which may be interconnect dielectric disposed between conductive components of the circuitries of the CMOS substrate as described above.

With reference to FIG. 7B, in a first step of the process, the layer of dielectric 704 may be etched to create via 706. Via 706 provides a cavity for forming the spongy and porous electrode desired for a nanopore cell. As can be seen in FIG. 7B, via 706 includes sidewalls 707 formed by dielectric 704 which meet with a top surface 709 of conductive layer 702.

In a next step, shown in FIG. 7C, spongy and porous electrode layer 708 is deposited to fill via 706 that was created in FIG. 7B. The spongy and porous electrode layer 708 may be a layer of TiN grown and deposited in a manner to create rough, sparsely-spaced TiN columnar structures or columns of TiN crystals that provide a high specific surface area that can come in contact with an electrolyte. The spongy and porous TiN layer 708 can be deposited using different deposition techniques, including atomic layer deposition (ALD), chemical vapor deposition (CVD), physical vapor deposition (PVD) sputtering deposition, and the like. It will be understood that although TiN is described herein as a preferable electrode material, other suitable porous electrode material may be used.

In a further step, with reference to FIG. 7D, an excess portion of TiN layer 708 may be removed. For example, the excess TiN layer may be removed using chemical mechanical planarization (CMP) techniques. The remaining TiN deposited in via 706 forms a spongy and porous working electrode 710.

As described above, it may be desirable to protect porous working electrode 710 during further manufacturing steps. Specifically, as will be described below with reference to FIG. 7F, well formation eventually requires the forming of hydrophobic cladding over the porous electrode. However, if the hydrophobic cladding is formed directly on the porous electrode, residues of the hydrophobic cladding can be embedded in the gaps or cavities in the porous electrode. The organic residues can make the electrode surface less wettable and prevent fluid from contacting the surface of the electrode. As a result, the effective surface area can be reduced, causing a significant drop in the double layer capacitance at the electrolyte-electrode interface. To alleviate this problem, a thin buffer or sacrificial protective layer, such as an SiO₂ layer, can be formed on the electrode to protect the surface of the electrode prior to subsequent processing, such as the deposition of the polyimide layer. After the well is formed, the thin sacrificial layer can be removed from the top surface of the electrode. The thin buffer or sacrificial layer serves to protect the porous electrode layer from the hydrophobic layer during the well formation process. Accordingly, in a step prior to the well formation, with reference to FIG. 7E, a protective layer 712 may be deposited on top of working electrode 710. As described above, the protective layer 712 may be used as a buffer layer or sacrificial layer to protect working electrode 710 during manufacture so that it retains the desired wettability and capacitance for operation. As described in previously incorporated U.S. Patent Application Serial No. 15/920,158, protective layer 712 may be a dielectric material such as silicon oxide or a metal material such as titanium. In either case, the protective layer can be chosen so that it is selectably removable.

In a step shown in FIG. 7F, a hydrophobic cladding 714 may be formed and patterned to form a well 716 above working electrode 710 and protective layer 712. As can be seen in FIG. 7F, the presence of protective layer 712 avoids forming of the hydrophobic cladding 714 directly on working electrode 710. Once deposited and patterned, hydrophobic cladding 714 form the sidewalls of well 716. The bottom of well 716 shown in FIG. 7F initially includes protective layer 712.

As can be seen in FIG. 7G, once the hydrophobic cladding is formed and patterned and processing is otherwise finalized, protective layer 712 may be removed chemically to expose working electrode 710 to well 716. In particular, protective layer 712 may be removed by applying removal reagents containing hydrofluoric acid (HF), nitric acid, or any other reagents that suitably remove protective layer 712. As an example, the protective layer 712 may be removed by a wet etching process using hydrofluoric acid (HF). The resulting nanopore cell 750 has well 716 with exposed working electrode 710 and sidewalls formed by hydrophobic cladding 714.

An alternative process for constructing an electrochemical cell of a nanopore-based sequencing chip is provided in FIGS. 8A-8F. The process illustrated in FIGS. 8A-8F is similar to that illustrated in FIGS. 7A-7G, except that the working electrode and protective layer are patterned together using photolithography and dry etching rather than chemical mechanical planarization techniques as described in FIGS. 7A-7G. Specifically, FIG. 8A illustrates the same incoming material 700 including substrate 701, conductive layer 702, and dielectric layer 704 disposed thereon as in FIG. 7A, and FIG. 8B illustrates the same etching of dielectric 704 to create via 706 for forming the spongy and porous TiN electrode. As with FIG. 7B, via 706 includes sidewalls 707 formed by dielectric 704 which meet with a top surface 709 of conductive layer 702.

Next, as shown in FIG. 8C, spongy and porous TiN layer 708 is deposited to fill via 706 that was created in FIG. 8B, and protective layer 812 is deposited on porous TiN layer 708. Then, as shown in FIG. 8D, the porous TiN layer 708 and the protective layer 812 are patterned together to create the working electrode 810 with the patterned protective layer 813. As described above, pattern protective layer 813 serves to protect working electrode 810 during the subsequent well formation process described below with reference to FIG. 8E.

With reference to FIG. 8E, hydrophobic cladding 814 may be formed and patterned to form well 816 similar to the process shown in FIG. 7F. As described previously with reference to FIG. 7F, it can be seen in FIG. 8E that the presence of protective layer 813 avoids forming of the hydrophobic cladding 814 directly on working electrode 810. Once deposited and patterned, hydrophobic cladding 814 form the sidewalls of well 816 and the bottom of well 816 initially includes protective layer 813.

As can be seen in FIG. 8F, once the hydrophobic cladding is formed and patterned and processing is otherwise finalized, protective layer 813 may be removed chemically to expose working electrode 810 to well 816. In particular, protective layer 813 may be removed by applying removal reagents containing hydrofluoric acid (HF), nitric acid, or any other reagents that suitably remove protective layer 813. As an example, the protective layer 813 may be removed by a wet etching process using hydrofluoric acid (HF). The resulting nanopore cell 850 has well 816 with exposed working electrode 810 and sidewalls formed by hydrophobic cladding 814.

Although the processes described in FIGS. 7A-7G and 8A-8F above generally provide for suitable electrochemical cells for nanopore-based sequencing, some drawbacks have been observed related to the growth of the porous electrode material. Specifically, it has been observed that while the deposition of porous electrode material results in the growth of columns of the electrode material with the desired porous characteristics, such growth not only occurs upwards from the top of the conductive surface (i.e. top surface 709 depicted in FIGS. 7B and 8B) as intended, but also perpendicularly to this intended growth from the sidewalls (i.e. sidewalls 707 depicted in FIGS. 7B and 8B) of the deposition vias. As a result, the columns growing upwards from the top of the conductive surface and the columns growing sideways from the sidewalls of the deposition vias collide with each other and form seams that continue to propagate until the deposition of the TiN is complete.

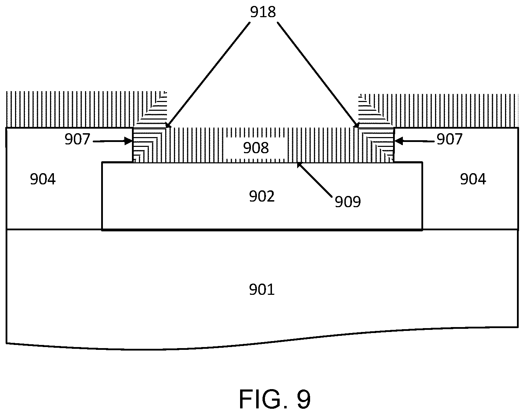

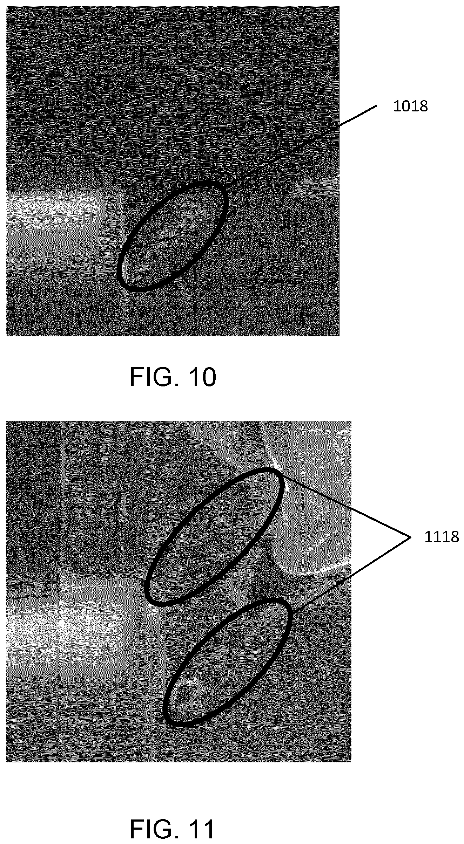

FIG. 9 illustrates seams that can form in working electrodes constructed using the processes illustrated in FIGS. 7A-7G and 8A-8F. As can be seen in FIG. 9, columns of TiN 908 grow both vertically from the top surface 909 of conductive layer 902 and horizontally from sidewalls 907 to form seams 918. Similar seams have been observed in examples that have been manufactured using processes similar to FIGS. 7A-7G and 8A-8F. For example, seams 1018 can be seen in FIG. 10, which is a micrograph illustrating a TiN electrode layer formed using the process illustrated in FIGS. 7A-7G, and seams 1118 can be seen in FIG. 11, which is a micrograph illustrating a TiN electrode layer formed using the process illustrated in FIGS. 8A-8F.

It will be understood (and can be seen in FIGS. 10 and 11) that the aforementioned seams that form in the non-planar vias ultimately result in unwanted voids which become weak points in the structure of the electrode layer. These voids and weak points may allow materials to seep through the electrode layer and interact with or attack other layers of the nanopore cell and drastically impact performance of the nanopore cell. In particular, removal reagents used for removing protective layers (such as HF or nitric acid containing reagents) as described in the processes above may seep through the voids created by the seams described. For example, these reagents may attack the interconnect dielectric layer (e.g., layer 704 described in FIGS. 7A-7G and 8A-8F), which may be used to separate conductive interconnects for a device. Ultimately, damage to these layers can lead to misalignment of well structures, shorting of electrodes, and cross talk between electrodes, all of which can severely impact performance of the nanopore cell.

### II. NANOPORE CELLS WITH SEAMLESS ELECTRODES

### A. Process for constructing seamless working electrode

In order to avoid the aforementioned seams and drawbacks associated therewith, methods are described for constructing nanopore cells with seamless working electrodes. In particular, FIGS. 12A-12F illustrate an embodiment of a process for constructing an electrochemical cell of a nanopore-based sequencing chip that includes a seamless working electrode.

As can be seen in FIG. 12A, incoming material 1200 may be similar to the incoming material 700 described previously with respect to FIGS. 7A and 8A, including a substrate 1201, a conductive layer 1202, and interconnect dielectric layer 1204 disposed thereon.

In a next step, as shown in FIG. 12B, planar electrode support layer 1205 may be formed by removing at least a portion of interconnect dielectric layer 1204. Planar electrode support layer 1205 includes both the conductive layer island 1202 and the surrounding interconnect layer 1204 that remains after removal of the portion thereof. Accordingly, planar electrode support layer 1205 has a planar electrode support surface 1203 that includes both the top surface of the conductive layer island 1202 and the top surfaces of the surrounding interconnect layer 1204 that remains after removal. The removal of dielectric layer 1204 may be done by blanket etching dielectric layer 1204 to expose the island of conductive layer 1202. In contrast with previously described processes, there are no vias with sidewalls in which electrode material is deposited. Rather, the electrode material will be deposited on a fully planar electrode support surface 1203.

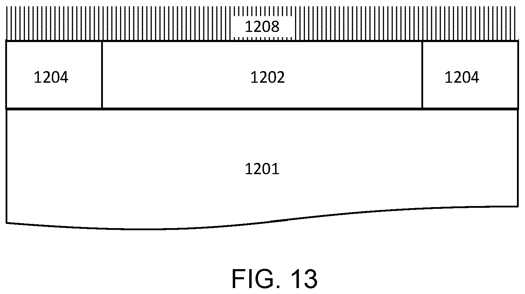

In a next step, depicted in FIG. 12C, porous electrode material 1208, such as TiN, is deposited on planar electrode support surface 1203. As can be seen in FIG. 12C, there is no sidewall of a via for perpendicular columnar growth of the porous electrode material, so there are no seams created in the porous electrode material. Thus, the porous electrode material 1208 that is deposited forms into uniform seamless columns of porous electrode material. These uniform seamless columns are further illustrated in FIG. 13, which illustrates the seamless electrode layer constructed using the process illustrated in FIGS. 12A-12C. Once deposited, and as can also be seen in FIG. 12C, seamless porous electrode material 1is protected by depositing protective layer 1212 thereon. As described above, protective layer 1212 may be comprised of a dielectric material or a metal material. Examples of a protective dielectric that can be used include silicon oxide, titanium oxide, hafnium oxide, zirconium oxide. Other suitable dielectric materials for use in embodiments of the present invention (e. g., protective layer 1212) include, without limitation, oxides, nitrides (e.g., silicon mononitride or SiN), silicon oxide, silicon oxynitride, metal oxides, metal nitrides, metal silicates, transition-metal oxides, transition-metal nitrides, transition metal-silicates, oxynitrides of metals, metal aluminates, zirconium oxide, zirconium silicate, zirconium aluminate, hafnium oxide, titanium oxide, or combinations thereof. Those of ordinary skill in the art will appreciate other dielectric materials that are suitable for use in the present invention. Examples of other materials that can be used as protective layers include titanium, aluminum, tantalum, tungsten, or nonporous titanium nitride. Those of ordinary skill in the art will appreciate other metal materials that are suitable for use in the present invention.

Next, as shown in FIG. 12D, the porous electrode layer 1208 and the protective layer 1212 are patterned together to create the patterned working electrode 1210 and patterned protective layer 1213. The TiN and protective layers may be patterned together using photolithography and dry etching, or other suitable processes used to pattern such materials. The resulting working electrode is an island of uniform columns of TiN without the undesirable seams previously described, with a protective layer disposed thereon.

In a further step, with reference to FIG. 12E, hydrophobic cladding 1214 is formed on electrode 1210 and protective layer 1213 and patterned to form well 1216 similar to the process shown in FIGS. 7F and 8E. As described previously with reference to FIG. 7F and 8E, the patterned hydrophobic cladding 1214 form the sidewalls of well 1216, and the bottom of well 1216 initially includes protective layer 1213 so as not to expose working electrode 1210. Examples of hydrophobic material that can be used include polyimides, epoxies, polybenzoxazoles (PBOs), benzocyclobutene (BCB). Those of ordinary skill in the art will appreciate other hydrophobic materials that are suitable for use in the present invention.

Next, with reference to FIG. 12F, once the manufacturing process is otherwise finalized, protective layer 1213 may be removed chemically using removal reagents, thus exposing working electrode 1210 to well 1216. As described above, protective layer 1213 may be removed chemically by removal reagents containing HF, nitric acid, other suitable reagents, and/or combinations thereof, depending on the material selected for protective layer 1212/1213. For example, if protective layer 1212/1213 is comprised of silicon dioxide or the like, buffered oxide etch (BOE) in concentrations ranging from 8:1 to 100:1 (NH4F:HF) may be used for removal. On the other hand, if protective layer 1212/1213 is comprised of a metal material, a diluted HF/nitric acid mixture with concentrations ranging from 10:1:1 and 500:1:1 (H2O:HF:HNO3) may be used for removal. Those of ordinary skill in the art will appreciate other reagents that are suitable for use in the present invention. Depending on the protective material and removal reagent, different processes may be used to remove the protective material. In some embodiments, protective layer 1213 may be removed by a wet etching process using any of the aforementioned reagents, but those of ordinary skill in the art will appreciate other processes that are suitable for removal in the present invention. In contrast with the previously described methods, since the electrode 1210 is formed with uniform seamless columns of TiN, there is much less chance of any removal reagents seeping through to attack other layers such as dielectric 1204.

FIG. 16 is a flowchart illustrating a method for forming a nanopore cell with a seamless electrode. Method 1600 includes, at step 1610, providing a device structure that includes a conductive layer disposed on a top portion of a substrate and an interconnect dielectric layer overlying the conductive layer. More details are provided in the description in connection with FIG. 12A. At step 1620, a portion of the interconnect dielectric is removed to form a planar electrode support surface including an exposed island of the conductive layer surrounded by the remaining portion of the interconnect dielectric layer. More details are provided in the description in connection with FIG. 12B. At step 1630, a porous electrode material is deposited on the planar electrode support surface to form a seamless porous electrode layer made of columns of the porous electrode material. More details are provided in the description in connection with FIG. 12C and 13. At step 1640, a protective layer is deposited on the seamless porous electrode layer. More details are provided in the description in connection with FIG. 12C. At step 1650, the seamless porous electrode layer and protective layer are patterned to form a working electrode island. More details are provided in the description in connection with FIG. 12D. At step 1660, a hydrophobic cladding is deposited and patterned to form the sidewalls of a well of the nanopore cell. More details are provided in the description in connection with FIG. 12E. At step 1670, a portion of the protective layer is removed to expose the porous electrode layer to the well of the nanopore cell. More details are provided in the description in connection with FIG. 12F.

### B. Nanopore cell with seamless working electrode

FIG. 12F illustrates a nanopore cell 1250 that includes a substrate 1201, an electrode support layer 1205 overlying a top portion of substrate 1201, and well 1216. Electrode support includes conductive layer island 1202 which is surrounded by interconnect dielectric layer 1204, and a planar top surface 1203 formed by the conductive layer island 1202 and the interconnect dielectric layer 1204. Well 1216 includes seamless porous working electrode island 1210, hydrophobic cladding 1214 surrounding the seamless porous working electrode island 1210 and patterned to form sidewalls of well 1216 and a cavity formed by the hydrophobic cladding 1214 and seamless porous working electrode island 1210. Seamless porous working electrode island 1210 is disposed on planar top surface 1203 of planar electrode support layer 1205 and is made of columns of a porous electrode material.

FIGS. 14A and 14B are top and perspective views micrographs, respectively, of an example of an array of seamless electrodes formed by the method of FIGS. 12A-12E, and FIG. 15 is a SEM (scanning electron microscope) image illustrating an example of a nanopore device with a seamless TiN electrode layer 1510 formed by the method of FIGS. 12A-12E. As can be seen in each of these figures, the columns of electrode material have the desired porosity and uniformity, without any visible seams that may serve as voids or weak points allowing removal reagents to seep through. Thus, the electrode material as produced using the method of FIGS. 12A-12E may be better configured to prevent damage to other components of the nanopore well.

In the preceding description, for the purposes of explanation, numerous details have been set forth in order to provide an understanding of various embodiments of the present technology. It will be apparent to one skilled in the art, however, that certain embodiments may be practiced without some of these details, or with additional details.

Where a range of values is provided, it is understood that each intervening value, to the tenth of the unit of the lower limit unless the context clearly dictates otherwise, between the upper and lower limits of that range, is also specifically disclosed. Each smaller range between any stated value or intervening value in a stated range and any other stated or intervening value in that stated range is encompassed. The upper and lower limits of these smaller ranges may independently be included or excluded in the range, and each range where either, neither, or both limits are included in the smaller ranges is also encompassed within the invention, subject to any specifically excluded limit in the stated range. Where the stated range includes one or both of the limits, ranges excluding either or both of those included limits are also included.

As used herein and in the appended claims, the singular forms "a", "an", and "the" include plural referents unless the context clearly dictates otherwise. Thus, for example, reference to "a method" includes a plurality of such methods. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Several embodiments of the invention are described above. For example, even though a polyimide layer is used as an example of a hydrophobic material for well formation in the above description, other organic material having hydrophobic surface properties, such as CYTOP, which is an amorphous fluoropolymer, may also be used in other embodiments. Moreover, besides silicon oxide, other dielectric materials having proper etch selectivity and process compatibility can also be used to form the sacrificial layer, for example, silicon nitride, zirconium oxide, and hafnium oxide, etc. Additionally, a number of well-known processes and elements have not been described in order to avoid unnecessarily obscuring the present invention. Additionally, details of any specific embodiment may not always be present in variations of that embodiment or may be added to other embodiments.

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

Although the terms "first" and "second" may be used herein to describe various features/elements (including steps), these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present invention.

Throughout this specification and the claims which follow, unless the context requires otherwise, the word "comprise", and variations such as "comprises" and "comprising" means various components can be co-jointly employed in the methods and articles (e.g., compositions and apparatuses including device and methods). For example, the term "comprising" will be understood to imply the inclusion of any stated elements or steps but not the exclusion of any other elements or steps.

As used herein in the specification and claims, including as used in the examples and unless otherwise expressly specified, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/- 0.1% of the stated value (or range of values), +/- 1% of the stated value (or range of values), +/- 2% of the stated value (or range of values), +/- 5% of the stated value (or range of values), +/- 10% of the stated value (or range of values), etc. Any numerical values given herein should also be understood to include about or approximately that value, unless the context indicates otherwise. For example, if the value "10" is disclosed, then "about 10" is also disclosed. Any numerical range recited herein is intended to include all sub-ranges subsumed therein. It is also understood that when a value is disclosed that "less than or equal to" the value, "greater than or equal to the value" and possible ranges between values are also disclosed, as appropriately understood by the skilled artisan. For example, if the value "X" is disclosed the "less than or equal to X" as well as "greater than or equal to X" (e.g., where X is a numerical value) is also disclosed. It is also understood that the throughout the application, data is provided in a number of different formats, and that this data, represents endpoints and starting points, and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

The examples and illustrations included herein show, by way of illustration and not of limitation, specific embodiments in which the subject matter may be practiced. This disclosure is intended to cover any and all adaptations or variations of various embodiments.

## Claims

1. A method for forming a nanopore cell (1250), comprising:
providing a device structure comprising:
a conductive layer (1202) disposed on a top portion of a substrate (1201); and
an interconnect dielectric layer (1204) overlying the conductive layer;
removing a portion of the interconnect dielectric layer to form a planar electrode support surface (1203) comprising an exposed island of the conductive layer surrounded by a remaining portion of the interconnect dielectric layer;
depositing a porous electrode material (1208) on the planar electrode support surface to form a seamless porous electrode layer comprising columns of the porous electrode material;
depositing a protective layer (1212) on the seamless porous electrode layer;
patterning the seamless porous electrode layer and the protective layer to a form a working electrode island (1210);
depositing and patterning a hydrophobic cladding (1214) on the working electrode island to form the sidewalls of a well (1216) of the nanopore cell; and
removing at least a portion of the protective layer to expose the porous electrode layer to the well, wherein the exposed porous electrode layer forms at least a portion of a bottom wall of the well of the nanopore cell.

2. The method of claim 1, wherein removing a portion of the interconnect dielectric comprises blanket etching a portion of the interconnect dielectric.

3. The method of claim 1, wherein removing at least a portion of the protective layer to expose the porous electrode layer comprises applying removal reagents to the protective layer.

4. The method of claim 3, wherein the removal reagents are applied using a wet etching process.

5. The method of claim 3, wherein the removal reagents are applied to the protective layer without damaging the interconnect dielectric layer.

6. The method of any of claims 1-5, 8, 9, 12 and 14-17, wherein the seamless porous electrode layer and the protective layer are patterned using photolithography and dry etching.

7. A nanopore cell (1250), comprising:
a substrate (1201);
an electrode support layer (1205) overlying a top portion of the substrate, the electrode support layer comprising:
a conductive layer island (1202);
an interconnect dielectric layer (1204) surrounding the conductive layer island; and
a planar top surface (1203) formed by the conductive layer island and the interconnect dielectric layer; and
a well (1216), comprising:
a seamless working electrode island (1210) disposed on the planar top surface of the electrode support layer, the seamless working electrode island comprising columns of a porous electrode material;
hydrophobic cladding (1214) surrounding the seamless working electrode island and patterned to form sidewalls of the well;
a cavity formed by the hydrophobic cladding and the seamless working electrode island.

8. The method of claim 1 or the nanopore cell of claim 7, wherein the porous electrode material comprises porous TiN (titanium nitride).

9. The method of claim 1 or the nanopore cell of claim 7, wherein the porous electrode material comprises a ruthenium containing material.

10. The nanopore cell of claim 7, wherein the seamless working electrode island further comprises a protective layer disposed on the columns of porous electrode material, wherein the protective layer is configured to be selectably removable to expose the porous electrode material to the cavity.

11. The nanopore cell of claim 10, wherein the protective layer is configured to be removed by application of removal reagents.

12. The method of claim 3 or the nanopore cell of claim 11, wherein the removal reagents comprise hydrofluoric acid.

13. The nanopore cell of claim 11, wherein the columns of porous electrode material are configured to prevent the removal reagents from damaging the electrode support layer.

14. The method of claim 1 or the nanopore cell of claim 10, wherein the protective layer is comprised of a dielectric material.

15. The method of claim 1 or the nanopore cell of claim 14, wherein the protective layer is comprised of silicon oxide.

16. The method of claim 1 or the nanopore cell of claim 10, wherein the protective layer is comprised of a metal material.

17. The method of claim 1 or the nanopore cell of claim 16, wherein the protective layer is comprised of titanium.

18. The nanopore cell of any one of claims 7-17, wherein the conductive layer island comprises aluminum.

19. The nanopore cell of any one of claims 7-17, wherein the interconnect dielectric comprises silicon oxide.

20. The nanopore cell of any one of claims 7-17, wherein the hydrophobic cladding comprises polyimide.

## Patentansprüche

1. Verfahren zum Bilden einer Nanoporenzelle (1250), umfassend:
Bereitstellen einer Vorrichtungskonstruktion, umfassend:
eine leitende Schicht (1202), die auf einem oberen Teil eines Substrats (1201) angeordnet ist; und
eine dielektrische Verbindungsschicht (1204), die über der leitenden Schicht liegt;
Entfernen eines Teils der dielektrischen Verbindungsschicht, um eine planare Elektrodenträgerfläche (1203) zu bilden, die eine freiliegende Insel der leitenden Schicht umfasst, die von einem verbleibenden Teil der dielektrischen Verbindungsschicht umschlossen ist;
Abscheiden eines porösen Elektrodenmaterials (1208) auf der planaren Elektrodenträgerfläche, um eine nahtlose poröse Elektrodenschicht zu bilden, die Säulen aus dem porösen Elektrodenmaterial umfasst;
Abscheiden einer Schutzschicht (1212) auf der nahtlosen porösen Elektrodenschicht;
Strukturieren der nahtlosen porösen Elektrodenschicht und der Schutzschicht, um eine Arbeitselektrodeninsel (1210) zu bilden;
Abscheiden und Strukturieren eines hydrophoben Überzugs (1214) auf der Arbeitselektrodeninsel, um die Seitenwände eines Wells (1216) der Nanoporenzelle zu bilden; und
Entfernen mindestens eines Teils der Schutzschicht, um die poröse Elektrodenschicht dem Well freizulegen, wobei die freigelegte poröse Elektrodenschicht mindestens einen Teil einer unteren Wand des Wells der Nanoporenzelle bildet.

2. Verfahren nach Anspruch 1, wobei das Entfernen eines Teils der dielektrischen Verbindungsschicht das Flächenätzen eines Teils der dielektrischen Verbindungsschicht umfasst.

3. Verfahren nach Anspruch 1, wobei das Entfernen mindestens eines Teils der Schutzschicht zum Freilegen der porösen Elektrodenschicht das Aufbringen von Entfernungsreagenzien auf die Schutzschicht umfasst.

4. Verfahren nach Anspruch 3, wobei die Entfernungsreagenzien unter Anwendung eines Nassätzprozesses aufgebracht werden.

5. Verfahren nach Anspruch 3, wobei die Entfernungsreagenzien auf die Schutzschicht aufgebracht werden, ohne dass die dielektrische Verbindungsschicht beschädigt wird.

6. Verfahren nach einem der Ansprüche 1-5, 8, 9, 12 und 14-17, wobei die nahtlose poröse Elektrodenschicht und die Schutzschicht unter Anwendung von Fotolithographie und Trockenätzen strukturiert werden.

7. Nanoporenzelle (1250), umfassend:
ein Substrat (1201);
eine Elektrodenträgerschicht (1205), die über einem oberen Teil des Substrats liegt, wobei die Elektrodenträgerschicht Folgendes umfasst:
eine Insel der leitenden Schicht (1202);
eine dielektrische Verbindungsschicht (1204), die die Insel der leitenden Schicht umschließt; und
eine planare obere Fläche (1203), die von der Insel der leitenden Schicht und der dielektrischen Verbindungsschicht gebildet wird; und
ein Well (1216), das Folgendes umfasst:
eine nahtlose Arbeitselektrodeninsel (1210), die auf der planaren oberen Fläche der Elektrodenträgerschicht angeordnet ist, wobei die nahtlose Arbeitselektrodeninsel Säulen aus einem porösen Elektrodenmaterial umfasst;
einen hydrophoben Überzug (1214), der die nahtlose Arbeitselektrodeninsel umschließt und so strukturiert ist, dass er Seitenwände des Wells bildet;
einen Hohlraum, der von dem hydrophoben Überzug und der nahtlosen Arbeitselektrodeninsel gebildet wird.

8. Verfahren nach Anspruch 1 oder Nanoporenzelle nach Anspruch 7, wobei das poröse Elektrodenmaterial poröses TiN (Titannitrid) umfasst.

9. Verfahren nach Anspruch 1 oder Nanoporenzelle nach Anspruch 7, wobei das poröse Elektrodenmaterial ein Ruthenium enthaltendes Material umfasst.

10. Nanoporenzelle nach Anspruch 7, wobei die nahtlose Arbeitselektrodeninsel ferner eine Schutzschicht umfasst, die auf den Säulen aus porösem Elektrodenmaterial angeordnet ist, wobei die Schutzschicht dafür ausgebildet ist, wählbar entfernt werden zu können, um das poröse Elektrodenmaterial dem Hohlraum freizulegen.

11. Nanoporenzelle nach Anspruch 10, wobei die Schutzschicht dafür ausgebildet ist, durch Aufbringen von Entfernungsreagenzien entfernt zu werden.

12. Verfahren nach Anspruch 3 oder Nanoporenzelle nach Anspruch 11, wobei die Entfernungsreagenzien Fluorwasserstoffsäure umfassen.

13. Nanoporenzelle nach Anspruch 11, wobei die Säulen aus porösem Elektrodenmaterial dafür ausgebildet sind, zu verhindern, dass die Entfernungsreagenzien die Elektrodenträgerschicht beschädigen.

14. Verfahren nach Anspruch 1 oder Nanoporenzelle nach Anspruch 10, wobei die Schutzschicht aus einem dielektrischen Material besteht.

15. Verfahren nach Anspruch 1 oder Nanoporenzelle nach Anspruch 14, wobei die Schutzschicht aus Siliciumoxid besteht.

16. Verfahren nach Anspruch 1 oder Nanoporenzelle nach Anspruch 10, wobei die Schutzschicht aus einem Metallmaterial besteht.

17. Verfahren nach Anspruch 1 oder Nanoporenzelle nach Anspruch 16, wobei die Schutzschicht aus Titan besteht.

18. Nanoporenzelle nach einem der Ansprüche 7-17, wobei die Insel der leitenden Schicht Aluminium umfasst.

19. Nanoporenzelle nach einem der Ansprüche 7-17, wobei die dielektrische Verbindungsschicht Siliciumoxid umfasst.

20. Nanoporenzelle nach einem der Ansprüche 7-17, wobei der hydrophobe Überzug Polyimid umfasst.

## Revendications

1. Procédé de formation d'une cellule à nanopores (1250), comprenant :
la fourniture d'une structure de dispositif comprenant :
une couche conductrice (1202) disposée sur une partie supérieure d'un substrat (1201) ; et
une couche diélectrique d'interconnexion (1204) recouvrant la couche conductrice ;
le retrait d'une partie de la couche diélectrique d'interconnexion pour former une surface de support d'électrode plane (1203) comprenant un îlot exposé de la couche conductrice entourée par une partie restante de la couche diélectrique d'interconnexion ;
le dépôt d'un matériau d'électrode poreux (1208) sur la surface de support d'électrode plane pour former une couche d'électrode poreuse sans discontinuité comprenant des colonnes du matériau d'électrode poreux ;
le dépôt d'une couche protectrice (1212) sur la couche d'électrode poreuse sans discontinuité ;
la structuration de la couche d'électrode poreuse sans discontinuité et de la couche protectrice pour former un îlot d'électrode de travail (1210) ;
le dépôt et la structuration d'un revêtement hydrophobe (1214) sur l'îlot d'électrode de travail pour former les parois latérales d'un puits (1216) de la cellule à nanopores ; et
le retrait d'au moins une partie de la couche protectrice pour exposer la couche d'électrode poreuse au puits, la couche d'électrode poreuse exposée formant au moins une partie d'une paroi inférieure du puits de la cellule à nanopores.

2. Procédé selon la revendication 1, dans lequel le retrait d'une partie du diélectrique d'interconnexion comprend la gravure pleine plaque d'une partie du diélectrique d'interconnexion.

3. Procédé selon la revendication 1, dans lequel le retrait d'au moins une partie de la couche protectrice pour exposer la couche d'électrode poreuse comprend l'application de réactifs de retrait sur la couche protectrice.

4. Procédé selon la revendication 3, dans lequel les réactifs de retrait sont appliqués en utilisant un processus de gravure humide.

5. Procédé selon la revendication 3, dans lequel les réactifs de retrait sont appliqués sur la couche protectrice sans endommager la couche diélectrique d'interconnexion.

6. Procédé selon l'une quelconque des revendications 1 à 5, 8, 9, 12 et 14 à 17, dans lequel la couche d'électrode poreuse sans discontinuité et la couche protectrice sont structurées en utilisant la photolithographie et la gravure sèche.

7. Cellule à nanopores (1250), comprenant :
un substrat (1201) ;
une couche de support d'électrode (1205) recouvrant une partie supérieure du substrat, la couche de support d'électrode comprenant :
un îlot de couche conductrice (1202) ;
une couche diélectrique d'interconnexion (1204) entourant l'îlot de couche conductrice ; et
une surface supérieure plane (1203) formée par l'îlot de couche conductrice et la couche diélectrique d'interconnexion ; et
un puits (1216), comprenant :
un îlot d'électrode de travail sans discontinuité (1210) disposé sur la surface supérieure plane de la couche de support d'électrode, l'îlot d'électrode de travail sans discontinuité comprenant des colonnes de matériau d'électrode poreux ;
un revêtement hydrophobe (1214) entourant l'îlot d'électrode de travail sans discontinuité et structuré pour former des parois latérales du puits ;
une cavité formée par le revêtement hydrophobe et l'îlot d'électrode de travail sans discontinuité.

8. Procédé selon la revendication 1 ou cellule à nanopores selon la revendication 7, dans lesquels le matériau d'électrode poreux comprend du TiN (nitrure de titane) poreux.

9. Procédé selon la revendication 1 ou cellule à nanopores selon la revendication 7, dans lesquels le matériau d'électrode poreux comprend un matériau contenant du ruthénium.

10. Cellule à nanopores selon la revendication 7, dans laquelle l'îlot d'électrode de travail sans discontinuité comprend en outre une couche protectrice disposée sur les colonnes de matériau d'électrode poreux, dans laquelle la couche protectrice est conçue pour pouvoir être sélectivement retirée afin d'exposer le matériau d'électrode poreux à la cavité.

11. Cellule à nanopores selon la revendication 10, dans laquelle la couche protectrice est conçue pour être retirée par application de réactifs de retrait.

12. Procédé selon la revendication 3 ou cellule à nanopores selon la revendication 11, dans lesquels les réactifs de retrait comprennent l'acide fluorhydrique.

13. Cellule à nanopores selon la revendication 11, dans laquelle les colonnes de matériau d'électrode poreux sont conçues pour empêcher les réactifs de retrait d'endommager la couche de support d'électrode.

14. Procédé selon la revendication 1 ou cellule à nanopores selon la revendication 10, dans lesquels la couche protectrice est constituée d'un matériau diélectrique.

15. Procédé selon la revendication 1 ou cellule à nanopores selon la revendication 14, dans lesquels la couche protectrice est constituée d'oxyde de silicium.

16. Procédé selon la revendication 1 ou cellule à nanopores selon la revendication 10, dans lesquels la couche protectrice est constituée d'un matériau métallique.

17. Procédé selon la revendication 1 ou cellule à nanopores selon la revendication 16, dans lesquels la couche protectrice est constituée de titane.

18. Cellule à nanopores selon l'une quelconque des revendications 7 à 17, dans laquelle l'îlot de couche conductrice comprend de l'aluminium.

19. Cellule à nanopores selon l'une quelconque des revendications 7 à 17, dans laquelle le diélectrique d'interconnexion comprend de l'oxyde de silicium.

20. Cellule à nanopores selon l'une quelconque des revendications 7 à 17, dans laquelle le revêtement hydrophone comprend du polyimide.
